# FASCICULE DE BREVET EUROPEEN

(11) **EP 2 953 925 B1**
(45) Date de publication et mention de la délivrance du brevet: **31.01.2018**
(21) Numéro de dépôt: 14706908.2
(22) Date de dépôt: 10.02.2014
(51) Int. Cl.: C07C 227/04

(54) **PROCÉDÉ DE SYNTHÈSE D'AMINOACIDE PAR METATHÈSE, HYDROLYSE PUIS HYDROGÉNATION**
VERFAHREN ZUR SYNTHESE VON AMINOSÄURE DURCH METATHESE, HYDROLYSE UND DANN HYDRIERUNG
METHOD OF SYNTHESISING AMINO ACID BY METATHESIS, HYDROLYSIS, THEN HYDROGENATION

(30) Priorité: 08.02.2013 FR 1351104
(43) Date de publication de la demande: 16.12.2015
(73) Titulaire: Arkema France, 92700 Colombes (FR)
(72) Inventeur: DUBOIS, Jean-Luc, F-69390 Millery (FR); COUTURIER, Jean-Luc, F-69006 Lyon (FR)
(74) Mandataire: Hérard, Elise
(86) Numéro de dépôt international: PCT/FR2014/050249
(87) Numéro de publication internationale: WO 2014/122412

(56) Documents cités:
- WO-A1-2010/055273
- RALUCA MALACEA ET AL: "Renewable materials as precursors of linear nitrile-acid derivatives via cross-metathesis of fatty esters and acids with acrylonitrile and fumaronitrile", GREEN CHEMISTRY, ROYAL SOCIETY OF CHEMISTRY, CAMBRIDGE, GB, vol. 11, no. 2, 1 janvier 2009 (2009-01-01), pages 152-155, XP008127125, ISSN: 1463-9262, DOI: 10.1039/B816917A [extrait le 2008-11-19]

## Description

### DOMAINE DE L'INVENTION

L'invention a pour objet un procédé de synthèse d'aminoacide de haute pureté à partir d'esters ou de nitriles gras insaturés impliquant une étape de métathèse.

### ARRIERE PLAN TECHNIQUE

Plusieurs voies de synthèse d'aminoacide à partir d'acides, esters ou nitriles gras insaturés impliquant une étape de métathèse ont déjà été testées. Cependant aucun des enchaînements de réactions testés ne donne vraiment satisfaction. Selon une première voie testée, la métathèse croisée suivie d'hydrogénation produit un aminoester présentant de nombreuses impuretés. Et s'il comporte plus de 9 atomes de carbone, l'aminoester s'avère très difficile à purifier, avec notamment un rendement de distillation bien trop insuffisant pour pouvoir envisager une application industrielle.

Selon une deuxième voie testée, la métathèse croisée suivie d'hydrogénation puis d'hydrolyse de l'aminoester obtenu dans le but de produire un aminoacide s'avère en pratique irréalisable, tant il est difficile d'hydrolyser un produit aussi réactif que l'aminoester puisqu'il a plutôt tendance à polymériser dans les conditions d'hydrolyse.

Selon encore une autre voie testée, on s'est rendu compte que la métathèse croisée faisant intervenir un acide, tel qu'un acide gras ou l'acide acrylique par exemple, donne de si faibles rendements en nitrile-acide (<60%), que même suivi d'hydrogénation, ce type de métathèse croisée ne conduit pas à un aminoacide de façon simple.

Par ailleurs les catalyseurs de métathèse et/ou d'hydrogénation ont tendance à entraîner la formation parasite d'amines secondaires parallèlement à la formation de l'amine primaire qu'est l'aminoacide visé. Le document WO2010/055273 concerne un procédé de synthèse d'un oméga-amino acide ou ester à partir d'un acide ou ester gras mono-insaturé. Si l'aminoester à 9 carbones sur la chaine linéaire est facilement purifiable par distillation, par exemple selon le procédé de Pechiney décrit dans le document de brevet FR1087798, ce n'est pas le cas des aminoesters plus lourds dont la chaine carbonée principale comporte plus de 9 atomes de carbones. Les procédés existants, notamment le procédé précité, ne permettent pas de résoudre le problème de purification pour les aminoesters à chaine longue à plus de 9 carbones.

Enfin la présence dans un aminoacide ou aminoester d'impuretés même en quantité apparemment faible de l'ordre de 0,5 à 1 %, rend très compliquée voire impossible l'utilisation de ces aminoacides ou aminoesters directement comme monomères de polymérisation ; ceci notamment à cause :
- des réactions de N-alkylation,
- des faibles degrés de polymérisation (DP) obtenus, qui ne dépassent pas 30 par exemple,

En particulier, la présence d'impuretés, telles que les amines secondaires, induit ces limitations de DP mais aussi :
- des problèmes de pollution du produit polymérisé,
- une coloration du produit polymérisé induite par les impuretés (aminés secondaires, mais aussi cendres : Nickel, sels minéraux, etc) présentes dans les monomères,
- ou encore des problèmes d'encrassement du dispositif de polymérisation.

La présente invention a donc pour but la synthèse d'aminoacide de haute pureté dont la teneur en impuretés, notamment en amines secondaires, est inférieure à 0,5% en poids, de préférence inférieure à 0,2% en poids, au moyen d'un procédé simple comprenant le moins d'étapes possibles, présentant un rendement élevé en aminoacide (>90%), et permettant une purification et donc une polymérisation aisée de l'aminoacide.

La Demanderesse a maintenant trouvé un procédé, qui selon un enchaînement d'étapes bien particulier, permet d'atteindre ce but.

### RESUME DE L'INVENTION

La présente invention a donc pour objet un procédé de synthèse d'un aminoacide à partir d'un composé I gras insaturé de formule :

R1-CH=CH-[(CH₂)_{q}-CH=CH]ₚ -(CH₂)ₙ-R2

dans laquelle :
R1 est H, un radical alkyle de 1 à 11 atomes de carbone comportant le cas échéant une fonction hydroxyle, ou (CH2)m-R4
m est un indice entier compris dans la gamme de 0 à 11,
n est un indice entier compris dans la gamme de 2 à 13,
p est un indice entier, p étant égal à 0, 1 ou 2,
q est un indice égal à 0 ou 1,
R2 est COOR5 ou CN,
   R4 est H ou R2
R5 est un radical alkyle de 1 à 11 atomes de carbone ou un radical comportant deux ou trois atomes de carbone porteur de une ou deux fonction(s) hydroxyle(s), ou encore le reste d'un di-glycéride ou d'un tri-glycéride dans lequel chaque acide gras dudit reste de glycéride est indifféremment saturé ou insaturé,
caractérisé en ce qu'il comprend au moins les étapes suivantes :
   - métathèse croisée avec un composé II court insaturé,
      l'un des composés I ou II comportant une fonction nitrile et l'autre de ces composés II ou I une fonction ester, de façon à obtenir et récupérer au moins un nitrile-ester mono-unsaturé (ci-après abrégé « NEU »),
   - hydrolyse du NEU en nitrile acide insaturé (ci-après abrégé « NAU »)
   - hydrogénation du NAU en aminoacide saturé (ci-après abrégé « AA »).
   - purification éventuelle de l'AA (étape optionnelle).

### DESCRIPTION DE MODES DE REALISATION DE L'INVENTION

Au sens de l'invention, la métathèse croisée (ci-après abrégé « mc ») est une réaction de métathèse entre un composé ester et un composé nitrile :
- soit entre un ester gras insaturé généralement issu de l'oléochimie avec un composé nitrile insaturé à chaine courte, tel que l'acrylonitrile,
- soit entre un composé nitrile gras insaturé généralement issu de l'oléochimie avec un composé ester insaturé à chaine courte, tel qu'un acrylate, et dans ce cas de préférence l'acrylate de méthyle.

Le procédé de l'invention a été développé en vue de l'exploitation de matières premières issues de sources naturelles renouvelables. On utilise donc de préférence comme « composé I » un ester gras insaturé ou un nitrile gras insaturé dérivé d'acide gras naturel. Mais le composé I peut tout aussi bien être choisi parmi des composés insaturés analogues obtenus par synthèse chimique.

La métathèse croisée est de préférence réalisée en présence d'un catalyseur de métathèse de type carbène de ruthénium comme décrit plus loin.

Comme composé I de départ utilisable dans le procédé de l'invention, on entend tout particulièrement un ester ou nitrile gras insaturé, de préférence d'origine naturelle, de formule I :

R1-CH=CH-[(CH₂)_{q}-CH=CH]ₚ-(CH₂)ₙ-R2

dans laquelle :
R1 est H, un radical alkyle de 1 à 11 atomes de carbone comportant le cas échéant une fonction hydroxyle, ou (CH₂)ₘ-R4
m est un indice entier compris dans la gamme de 0 à 11,
n est un indice entier compris dans la gamme de 2 à 13,
p est un indice entier, p étant égal à 0, 1 ou 2,
q est un indice égal à 0 ou 1,
R2 est COOR5 ou CN,
R4 est H ou R2
R5 est un radical alkyle de 1 à 11, de préférence de 1 à 5, de préférence de 1 à 4, atomes de carbone ou un radical comportant deux ou trois atomes de carbone porteur de une ou deux fonction(s) hydroxyle(s), ou encore le reste d'un di-glycéride ou d'un tri-glycéride dans lequel chaque acide gras dudit reste de glycéride est indifféremment saturé ou insaturé.

Par composé « gras » au sens de l'invention, on entend un composé comportant de 8 à 57 (notamment dans le cas d'un triglycéride) atomes de carbone, de préférence de 8 à 36 atomes de carbone, de préférence de 10 et 24 atomes de carbone, et comportant de préférence 10 ou 11 atomes de carbone.

Le composé I comporte au moins une insaturation, c'est-à-dire une double liaison C=C. Chaque double liaison C=C du composé I peut être en conformation cis ou trans. L'insaturation est localisée en position x par rapport au groupement ester ou nitrile, cette position est désignée par convention « delta x »). Ceci permet de déterminer la formule de l'ω-amino-acide final obtenu selon le procédé de l'invention.

Le composé de formule I R₁-CH=CH-[(CH₂)_{q}-CH=CH]ₚ-(CH₂)ₙ-R₂ est avantageusement choisi parmi les esters d'acides gras (y-compris les mono-, di-, et tri-glicérides) ou les nitriles dérivés d'acides gras, d'origine végétale ou animale (y compris ceux issus d'algues naturelles).

On peut citer à titre d'exemples d'acides gras, les acides en C10, les acides obtusilique (cis-4-décénoïque) et caproléique (9-décénoïque), les acides en C12, les acides lauroléique (cis-5-dodécénoïque) et lindérique (cis-4-dodécénoïque), les acides en C14, les acides myristoléique (cis-9-tétradécénoïque), physétérique (cis-5-tetradécénoïque) et tsuzuique (cis-4-tetradécénoïque), l'acide en C16, l'acide palmitoléique (cis-9- hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoïque) et ricinoléique (12-hydroxy--cis-9-octadécénoïque), les acides en C20, les acides gadoléique (cis-9-eicosénoïque), gondoïque (cis-11-eicosénoïque), l'acide cis-5-eicosènoïque et l'acide lesquérolique (14-hydroxy-cis-11-eicosénoïque), les acides en C22, les acides cétoléique (cis-11-docosénoïque) et érucique (cis-13-docosénoïque) ainsi que les acides polyinsaturés linoléique et linolénique.

Ces divers acides sont issus des huiles végétales extraites de diverses plantes oléagineuses telles que le tournesol, le colza, le ricin, le lesquerella, l'olive, le soja, le palmier, l'avocat, l'argousier, la coriandre, le céleri, l'aneth, la carotte, le fenouil, le Limnanthes Alba (meadowfoam), le carthame, la caméline, le Jatropha.

Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins. Cependant pour des raisons techniques de conduite de la réaction de métathèse, il est souvent préférable de modifier cet ester ou nitrile dérivé d'acide gras en le soumettant à une réaction préalable comprenant une éthénolyse, buténolyse ou propénolyse ou un craquage thermique (pyrolyse) permettant de conduire à un ester ou nitrile gras de formules telles que CH₂=CH-(CH₂)ₙ-R2, CH₃-CH=CH-(CH₂)ₙ-R2 ou CH₃-CH₂-CH=CH-(CH₂)ₙ-R2.

La métathèse croisée peut aussi bien être mis en oeuvre sur des corps gras omega insaturés dont la double liaison se trouve en fin de chaîne, que sur des corps gras dont la double liaison est interne, mais on utilise de préférence les corps gras omega insaturés. On utilise de préférence un composé I de départ mono-insaturé et oméga-insaturé, et de préférence le composé I est un ester ou un nitrile gras de formule CH₂=CH-(CH₂)ₙ-R2.

Selon un mode de réalisation avantageux du procédé de l'invention, on utilise un composé I dans lequel R2 est COOR5. De préférence dans ce cas, la métathèse croisée sur le composé I est réalisée avec un composé II nitrile insaturé à chaine courte (chaine principale comprenant moins de 8 carbones).

Le composé II nitrile insaturé à chaine courte est avantageusement choisi parmi : l'acrylonitrile, le fumaronitrile, le 2-butènenitrile, le 1-butènenitrile, le 2-pentènenitrile, le 3-pentène nitrile, le 4-pentènenitrile, le 1-pentènenitrile, et leurs mélanges. De préférence, le composé II est l'acrylonitrile.

On forme ainsi un nitrile-ester insaturé (ci après abrégé « NEU »), et un coproduit diester à longue chaîne.

De préférence, le composé I insaturé est un ester gras mono-insaturé oméga-insaturé de formule CH₂=CH-(CH₂)ₙ-COOR5.

R5 est un radical alkyle comprenant de 1 à 11, de préférence de 1 à 5 atomes de carbone, de préférence de 1 à 2 atomes de carbone.

Selon un mode de réalisation avantageux du procédé de l'invention, le composé I ester gras oméga-insaturé, y compris lorsque cet ester gras omega insaturé est présent sous la forme de mono-, di- ou tri-glycérides, est mis en oeuvre avec un composé II nitrile court insaturé, tel que l'acrylonitrile, en présence d'un catalyseur de métathèse, de préférence en ajoutant de manière continue le catalyseur. Pendant cette première étape, où l'acrylonitrile réagit, de l'éthylène se dégage de la réaction, et un mélange de nitrile-ester et de diester est formé.

Selon un autre mode de réalisation avantageux du procédé de l'invention, on utilise un composé I dans lequel R2 est CN.

De préférence, le composé I insaturé est un nitrile gras oméga-insaturé de formule CH₂=CH-(CH₂)ₙ-CN.

De préférence dans ce cas la métathèse croisée sur le composé I nitrile gras est réalisée avec un composé II ester insaturé à chaine courte (chaine principale comprenant moins de 8 carbones), de préférence un acrylate, tel que l'acrylate de méthyle. Dans ce cas, pendant cette étape de métathèse croisée, où l'acrylate réagit, de l'éthylène se dégage de la réaction, et un mélange de nitrile-ester et de dinitrile se forme.

Le composé II ester insaturé à chaine courte est avantageusement choisi parmi les composés de formule : R6-HC=CH-(CH₂)ₙ-COOR7 dans laquelle :
n est 0 ou 1 ; R6 est CH₃ ou H ; R7 est Me (métyle), Et (étyle) ou Bu (butyle). De préférence, le composé II est l'acrylate de méthyle.

La réaction de métathèse est conduite en présence d'au moins un catalyseur de métathèse. Ces catalyseurs sont bien connus et il en existe toute une gamme. On peut citer par exemple les complexes au tungstène développés par Schrock et al (J. Am. Chem. Soc. 108:2771, 1986) ou Basset et al. (Angew. Chem., Ed. Engl. 31:628, 1992). Plus récemment, sont apparus les catalyseurs dits de Grubbs (voir Grubbs et al., Angew. Chem., Ed. Engl. 34 :2039, 1995 et Organic Letters 1:953, 1999) qui sont des complexes ruthénium-benzylidène opérant en catalyse homogène. D'autres travaux ont été conduits pour la réalisation de catalyseurs immobilisés, c'est-à-dire de catalyseurs dont le principe actif est celui du catalyseur homogène, notamment les complexes ruthénium-carbène, immobilisés sur un support inactif.

Le procédé selon l'invention utilise avantageusement au moins un catalyseur de métathèse de type ruthénium-carbène. Ledit catalyseur ruthénium-carbène est choisi de préférence parmi les catalyseurs chargés ou non-chargés de formule générale :

(X₁)ₐ (X₂)_{b}Ru(carbène C) (L₁)_{c}(L₂)_{d}(L₃)ₑ

dans laquelle :
- a, b, c, d et e sont des nombres entiers, identiques ou différents, avec a et b égaux à 0, 1 ou 2 ; c, d et e égaux à 0, 1, 2, 3 ou 4;
- X₁ et X₂, identiques ou différents, représentent chacun un ligand mono-ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés ; X₁ ou X₂ peuvent être liés à L₁ ou L₂ ou au carbène C de façon à former un ligand bidenté ou chélate sur le ruthénium ; et
- L₁, L₂ et L₃ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; L₁, L₂ ou L₃ peuvent être liés au carbène C de façon à former un ligand bidenté ou chélate, ou tridenté.

Le carbène C est représenté par la formule générale : CR₁R₂ pour laquelle R₁ et R₂ sont des groupes identiques ou différents tels que l'hydrogène ou tout autre groupe hydrocarboné, fonctionnalisé ou non, de type saturé, insaturé, cyclique, aromatique, branché et/ou linéaire. A titre d'exemples, on pourra citer les complexes du ruthénium alkylidènes, benzylidène, benzylidène éther, ou cumylènes tels que les vinylidènes Ru=C=CHR ou allénylidènes Ru=C=C=CR₁R₂ ou indénylidènes.

Un groupe fonctionnel (permettant d'améliorer la rétention du complexe du ruthénium dans un liquide ionique) peut être greffé sur au moins l'un des ligands X₁, X₂, L₁, L₂, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, n°2, 2007, p.45-52.

Des exemples de tels catalyseurs sont les catalyseurs de Grubbs, les catalyseurs Hoveyda-Grubbs, les catalyseurs Piers-Grubbs, et autres catalyseurs de métathèse du même type, qu'ils soient dits de « 1^{ère} génération », « 2^{ème} génération » ou de « 3^{ème} génération ».

Les catalyseurs de Grubbs sont basés sur un atome de ruthénium entouré par 5 ligands :
- 2 ligands anioniques, tels que des halogénures ;
- 2 ligands donneurs d'électrons, tels que les tri-alkyl-phosphines, ou les carbènes N-hétérocycliques saturés (appelés ligands NHC) ;
- un groupement alkylidène, tels que des groupements méthylènes =CR₂ substitués ou non.

On classe ces catalyseurs de métathèse en deux catégories, suivant la nature de leurs ligands L donneurs d'électrons :
- ceux qui contiennent deux ligands phosphine (et pas de ligand NHC saturé), développés en premier, sont des catalyseurs de type 1^{ère} génération ;
- ceux qui contiennent un ligand NHC saturé (carbène hétérocyclique) sont des catalyseurs de type 2^{ème} génération.

Un type de catalyseur dit « Hoveyda-Grubbs » contient parmi les ligands donneurs d'électrons, un ligand chélatant benzylidène-éther, et soit une phosphine (1^{ère} génération) soit un ligand NHC saturé (2^{ème} génération), le plus souvent substitué par des phényles généralement substitués par des groupements mesityl (Mes) ou bien par des groupements isopropyl (iPr).

Un autre type de catalyseur dit « Piers-Grubbs », forme un complexe cationique à quatre ligands qui ne nécessite pas la dissociation d'un ligand avant la réaction. D'autres types de catalyseurs sont les catalyseurs « Umicore », « Zanan », « Grela ».

De manière générale, le choix du catalyseur dépend de la réaction considérée. Selon un mode de réalisation avantageux, le catalyseur est dépourvu de phosphine.

Des catalyseurs préférés sont les catalyseurs suivants :
(1) Le catalyseur désigné par « Hoveyda-Grubbs 2 », de formule suivante : 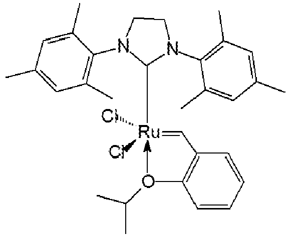
(2) Le catalyseur désigné par « M51 », de formule suivante : 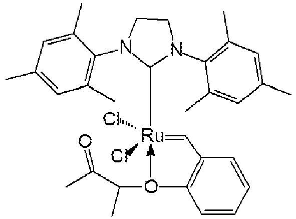
(3) Le catalyseur désigné par « M71-SIPr », de formule suivante : 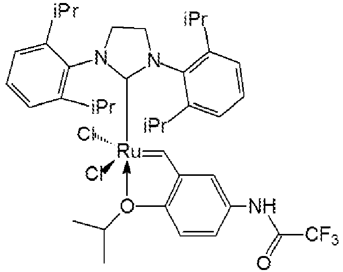
(4) Le catalyseur désigné par « M71-SIMes », de formule suivante : 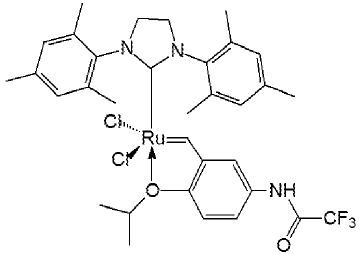
(5) Le catalyseur désigné par « M72-SIPr », de formule suivante : 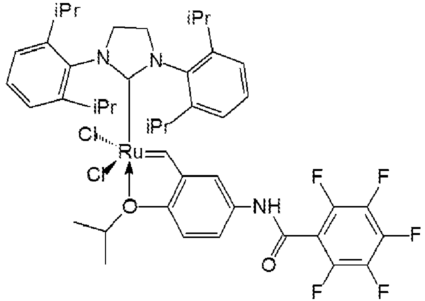
(6) Le catalyseur désigné par « M73-SIPr », de formule suivante : 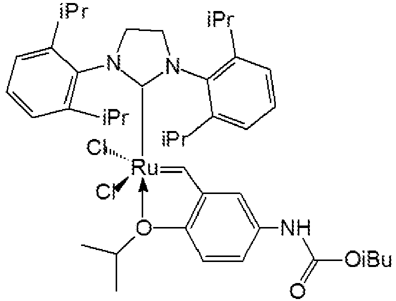
(7) Le catalyseur désigné par « M74-SIPr », de formule suivante : 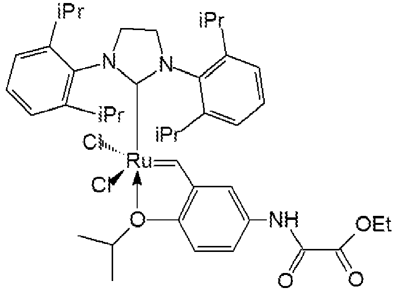
(8) Le catalyseur désigné par « Nitro-Grela-SIMes », de formule suivante : 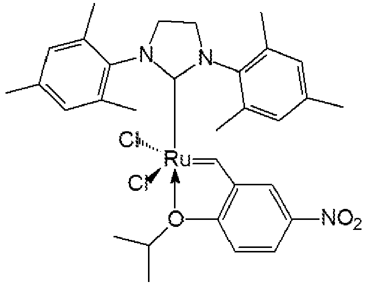
(9) Le catalyseur désigné par « Nitro-Grela-SIPr », de formule suivante : 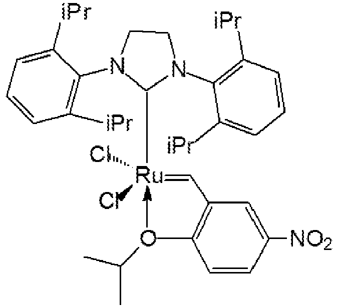
(10) Le catalyseur désigné par « Apeiron AS2034 », de formule suivante : 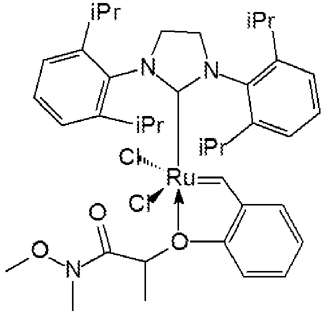
(11) Le catalyseur désigné par « Zannan 44-0082 (Strem) », de formule suivante : 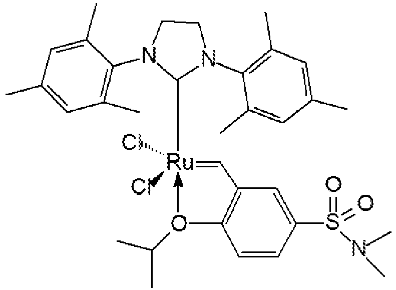
(12) Le catalyseur désigné par « M831 -SIPr », de formule suivante : 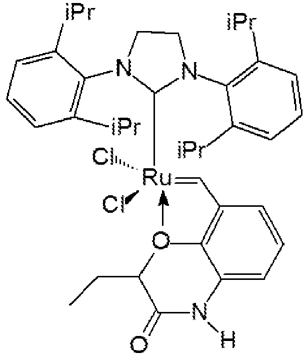
(13) Le catalyseur désigné par « M832-SIPr », de formule suivante : 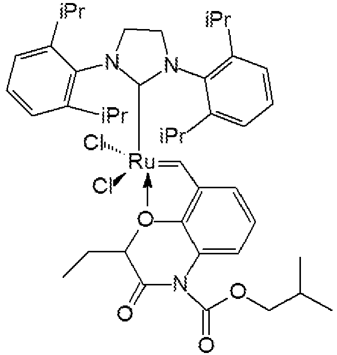
(14) Le catalyseur désigné par « M853-SIPr », de formule suivante : 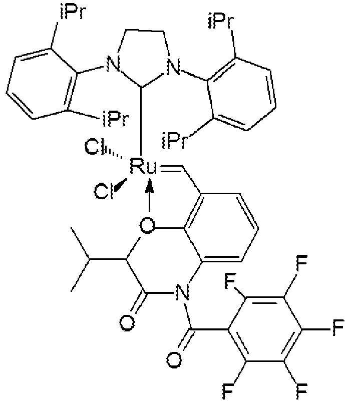
(15) Le catalyseur désigné par « M863-SIPr », de formule suivante : 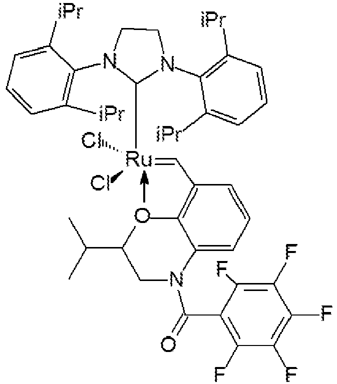
(16) Le catalyseur désigné par « Materia C711 », de formule suivante :

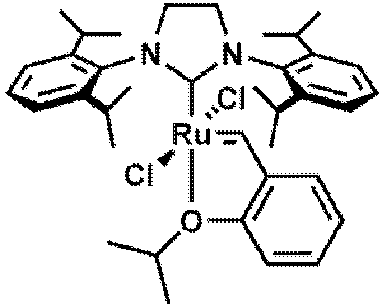

La réaction de métathèse est conduite de préférence en milieu liquide dans les conditions opératoires suivantes.

La température est généralement comprise dans la gamme de 20 à 160 °C et de préférence dans la gamme de 20 à 120 °C.

La pression est généralement comprise dans la gamme de 1 à 30 bars. La réaction est de préférence conduite à basse pression comprise dans la gamme de 1 à 10 bars et de façon plus préférée à la pression atmosphérique lorsque la température d'ébullition des réactifs mis en jeu le permet. En effet, si l'on vise toujours un dégagement d'oléfine légère, éthylène ou autre, il est avantageux de travailler à basse pression, pression atmosphérique de préférence.

La réaction peut être conduite sans solvant ou en présence d'au moins un solvant, tel que le toluène, les xylènes, le dichlorométhane, le diméthylcarbonate ou le diéthylcarbonate par exemple, et leurs mélanges.

A l'issue de la métathèse croisée, on obtient un NEU (le produit) et selon le cas un DE ou un DN (le coproduit). Bien que les étapes ultérieures du procédé puissent être réalisées directement sur ce mélange de produits issu de métathèse croisée, il est préférable d'isoler le NEU pour optimiser les rendements d'hydrolyse et d'hydrogénation du procédé de l'invention. De manière optionnelle, on sépare donc et récupère aisément le produit du coproduit de métathèse croisée, par distillation et/ou tout autre moyen bien connu de l'homme du métier. On peut notamment utiliser l'extraction liquide/liquide (par exemple pour séparer le solvant éventuel), la cristallisation (par exemple pour séparer le co-produit), l'adsorption (par exemple pour séparer le catalyseur).

Le procédé de l'invention comprend en outre, après l'étape de métathèse croisée décrite ci-dessus, l'hydrolyse du nitrile-ester insaturé NEU en nitrile-acide insaturé (ci-après « NAU »).

L'hydrolyse peut être réalisée selon différents procédés bien connus, et permettant d'hydrolyser la fonction ester sans hydrolyser la fonction nitrile du NEU. L'hydrolyse peut être conduite notamment comme décrit dans les documents « CEH - Natural Fatty Acids, by Michael P. Malveda, page 11 », à pression atmosphérique, par exemple en présence d'acides sulfurique et sulfonique ou sous pression, en mode batch ou discontinu, en présence d'un catalyseur tel que l'oxyde de zinc ou de magnésium et d'eau. On peut utiliser une catalyse acide ou basique. L'hydrolyse peut également être réalisée selon le procédé similaire à celui décrit page 314, III.1.3 de « Procédés de prétrochimie, tome 2, de A. Chauvell, G. Lefebvre, L. Castex ».

Parmi les procédés d'hydrolyse utilisables dans le procédé de l'invention, on peut notamment citer les 4 procédés suivants :
- hydrolyse basse température (15°C-60°C) en présence de soude (saponification) ;
- hydrolyse à température moyenne (60°C-120°C, de préférence de 60 à 100°C, ou mieux vers 80°C) en milieu solvant et sous catalyse acide ;
- hydrolyse haute température (120°C-300°C, de préférence 130°C-250°C) sous pression ;
- hydrolyse enzymatique généralement à basse température, mais plus largement à une température comprise dans la gamme de 10°C-100°C, de préférence de 10°C à 40°C.

Bien que l'étape ultérieure d'hydrogénation du procédé puisse être réalisée directement sur le produit directement issu de l'hydrolyse, il est préférable d'isoler le NAU pour optimiser le rendement d'hydrogénation du procédé de l'invention, notamment par cristallisation, distillation et/ou tout autre moyen bien connu de l'homme du métier.

Le procédé selon l'invention comprend en outre après l'étape d'hydrolyse, l'hydrogénation du NAU en AA.

L'hydrogénation est réalisée en présence d'au moins un catalyseur d'hydrogénation, selon différents procédés bien connus. Des exemples de catalyseurs appropriés sont ceux renfermant des éléments du Groupe VIII de la Classification Périodique des Eléments de Mendeleïev, à savoir le fer, le cobalt, le nickel et les métaux nobles, tels que le ruthénium, le rhodium, le palladium, l'osmium, l'iridium et le platine, seuls ou en mélange. Ces métaux peuvent éventuellement être dopés par de l'or. Les éléments du groupe VIII peuvent être présents dans le catalyseur sous la forme du métal ou de son oxyde. L'élément peut être utilisé sous la forme d'un catalyseur du type Raney. L'élément du groupe VIII présent sous forme de métal ou d'un composé peut être utilisé en mélange avec des quantités plus ou moins grandes d'un ou de plusieurs composés d'autres éléments.

Le catalyseur peut se trouver à l'état finement divisé, ou bien sous la forme de granulés, ou encore peut être déposé sur un support.

Des exemples de supports appropriés sont : la silice, la pierre ponce, le dioxyde de titane, le carbone, le charbon, le carbure de silicium ou l'alumine. Des exemples de catalyseurs préférés sont les suivants: catalyseurs au nickel, Nickel de Raney; catalyseurs au cobalt par exemple, le cobalt de Raney, et les oxydes de cobalt, en mélange avec d'autres composés tels que des phosphates, ainsi que les catalyseurs au platine. Les catalyseurs au platine peuvent être des catalyseurs renfermant du platine fixé sur du carbone Pt/C, fabriqué par des techniques bien connues et contenant du platine sous la forme de métal. On peut également utiliser le catalyseur d'Adams, qui est introduit dans le réacteur sous forme PtO₂ et qui est réduit in situ à l'état de platine métallique finement divisé.

La réaction peut être effectuée en phase liquide en mélangeant le matériau à hydrogéner avec un solvant. Quand on utilise des catalyseurs d'hydrogénation formés par des métaux précieux, par exemple Pt/C, le solvant peut renfermer judicieusement de l'acide acétique seul ou avec un autre liquide, de l'eau par exemple.

Quand on utilise du nickel Raney ou du cobalt Raney ou encore des catalyseurs formés par des oxydes des métaux du Groupe VIII fixés sur des supports, on peut citer à titre d'exemples de solvants appropriés les alcools, notamment les alcools inférieurs, par exemple ceux comportant de 1 à 8 atomes de carbone par molécule. Des exemples d'alcools inférieurs sont l'éthanol et les propanols, tels que le n-propanol et l'isopropanol.

L'alcool est de préférence utilisé en mélange avec de l'eau et il est particulièrement judicieux d'ajouter de l'ammoniac.

On ne constate que la proportion de sous-produits tels que des amines secondaires est diminuée par l'addition d'ammoniaque.

Le rapport molaire de l'ammoniac à la quantité totale de NAU initialement présente peut être par exemple de l'ordre de 1 à 1000, de préférence de 60 à 600, en particulier de 150 à 300.

La solution est mise en contact avec le catalyseur de toute manière convenable et on amène de l'hydrogène en contact avec le catalyseur et la solution.

Dans le cas où un catalyseur finement divisé est utilisé, il peut être dispersé dans la solution, et maintenu en dispersion par agitation de la solution.

Un moyen facile de mise en oeuvre de l'hydrogénation consiste à utiliser un lit de pastilles ou de granulés de catalyseur solide. On peut faire passer une solution de la matière à hydrogéner sur le catalyseur, par écoulement dans le même sens ou à contre-courant d'un courant d'hydrogène. Le lit peut être complètement immergé dans la solution, ou le lit peut être utilisé sous forme de lit à ruissellement. Dans ce cas, le lit est entouré par de l'hydrogène gazeux et la solution de la matière à hydrogéner peut s'infiltrer travers le lit.

La réaction peut être réalisée en discontinu par charges successives ou en continu.

La température et la pression optimales pour l'hydrogénation dépendent du catalyseur utilisé. Quand on utilise des catalyseurs à base de métaux nobles, par exemple Pt/C, on peut effectuer l'hydrogénation dans une gamme de conditions telles que : de 15°C à 100°C et sous une pression de 1 atm à 100 atm. Mais on obtient une plus grande sélectivité à des températures de 50 à 60 °C et des pressions de 60 à 80 atm. Quand on utilise des catalyseurs d'hydrogénation au nickel et au cobalt, on peut avantageusement utiliser des températures de 10 à 200°C et des pressions de 1 et 350 atm. La durée de la réaction dépend bien sûr des conditions de réaction et le temps de réaction peut aller de moins de 20 minutes à plus de 5 heures.

L'aminoacide saturé (abrégé « AA » dans la présente description) produit au cours de la réaction d'hydrogénation peut être récupéré de toute manière appropriée. Pour faciliter la récupération, il est judicieux, en particulier lorsqu'on opère sous pression, d'équiper le réacteur d'hydrogénation d'un filtre intérieur placé en-dessous du niveau du liquide et disposé de façon à empêcher toute obstruction (par le catalyseur par exemple) de la sortie servant à la récupération du produit, et de telle sorte que le filtre puisse être aisément nettoyé par passage d'hydrogène à travers le filtre.

Le produit est avantageusement soutiré à travers ce filtre, de sorte qu'il est recueilli sans catalyseur. Quand on utilise un mélange réactionnel chaud, le produit est soutiré de préférence à la température de réaction pour empêcher la séparation du produit dans le réacteur ou dans les conduits partant du réacteur. Le produit peut être prélevé par intermittence ou en continu, mais dans les deux cas, il est souhaitable de maintenir une atmosphère d'hydrogène sur le catalyseur afin d'éviter sa désactivation. L'aminoacide AA saturé peut être récupéré par refroidissement de la solution retirée du réacteur et / ou évaporation du solvant.

Selon un mode de réalisation avantageux du procédé de l'invention, l'hydrogénation est réalisée en présence d'au moins un catalyseur métallique choisi parmi le ruthénium, le rhodium, le palladium et le platine supporté sur un support carbure de silicium. La température d'hydrogénation est comprise dans la gamme de 10 à 300°C, de préférence de 20-200 °C, la pression est comprise dans la gamme de 1 bar à 300 bars. On utilise avantageusement comme solvant un mélange d'un alcool inférieur et d'eau, et de l'ammoniac présent dans le système réactionnel. Ces conditions d'hydrogénation permettent de diminuer voire de supprimer la production parallèle d' aminés secondaires.

On utilise donc de préférence un catalyseur d'hydrogénation dans lequel le ruthénium, le rhodium, le palladium, le platine ou un mélange de deux ou plusieurs de ces métaux (éventuellement dopés avec de l'or) sont chargés sur un support de carbure de silicium. La quantité des différents métaux précités chargée sur le support de carbure de silicium est avantageusement de 0,1 à 10% en poids, et de préférence de 0,2 à 1% en poids. La pureté du support de carbure de silicium est comprise dans la gamme de 70 à 100%. Le support peut comporter jusqu'à 30% d'impuretés telles que : silice, alumine, carbone, oxyde de fer, oxyde de calcium, oxyde de sodium, oxyde de potassium, etc. La pureté du support de carbure de silicium dans le procédé de l'invention est de préférence très élevée, et de préférence caractérisée par : l'absence de fer et l'absence (de couche) de silice superficielle, les 2 impuretés majeures du SiC. Pour le support de carbure de silicium, on utilise de préférence un matériau de porosité comprise dans la gamme de 20 à 60%, et dont la taille des pores est comprise dans la gamme de 1 à 200 µm, et il est préférable de le nettoyer avec de l'acide minéral et une solution aqueuse basique, avant la production du catalyseur, notamment pour éliminer les impuretés Fe et SiO2.

Les catalyseurs peuvent être préparés avec les méthodes connues pour le chargement des métaux sur un support. Par exemple, un catalyseur peut être préparé en dissolvant le trichlorure de ruthénium, le trichlorure de rhodium, le dichlorure de palladium, l'acide chloroplatinique ou un mélange de deux ou plusieurs de ceux-ci dans l'eau pour former une solution aqueuse, en la mettant en contact avec le support de carbure de silicium pour charger les divers composés métalliques mentionnés ci-dessus sur le support, puis en activant le catalyseur par des moyens de chauffage et de réduction dans un courant d'hydrogène. De plus, dans ce procédé de préparation comprenant l'imprégnation de différents composés métalliques mentionnés ci-dessus dans le support, le chauffage et la réduction dans un courant d'hydrogène, est de préférence répété plusieurs fois. D'autres procédés de préparation comprennent :
- des procédés dans lesquels des nitrates ou acétates de différents métaux sont dissous dans de l'eau ou un solvant organique tel que le benzène, le chloroforme ou l'alcool, ou bien dans un mélange d'eau et de solvant organique, et imprégnés dans le support, puis les différents composants métalliques qui ont été imprégnés dans le support sont traités thermiquement ou traités avec un alcalin et transformés en oxydes ou hydroxydes, et/ou
- des procédés dans lesquels le chauffage et la réduction sont réalisés dans un flux de gaz réducteur comprenant du formaldéhyde, de l'acide formique ou du méthanol, dans lequel une base est ajoutée à une solution d'acide formique ou formaldéhyde.

Les conditions de la réaction d'hydrogénation pour former l'aminoacide sont de préférence une température comprise dans la gamme de 10 à 200 °C, et de préférence de 70 à 130 °C, et une pression allant de 1 bar à 350 bars, et de préférence de 5 à 150 bars.

L'utilisation d'un solvant est préférée lors de l'hydrogénation du NAU à chaine longue (comprenant plus de 9 C) de l'invention. Des mélanges d'alcools inférieurs et d'eau peuvent être utilisés comme solvant. L'alcool inférieur est de préférence choisi parmi le n-propanol, l'isopropanol, le n-butanol et l'iso-butanol. Les proportions du mélange respectivement d'alcool inférieur et d'eau sont avantageusement comprises dans la gamme de 5:1 à 1:5 en volume.

La source d'hydrogène utilisée peut être de l'hydrogène pur ou de l'hydrogène dilué avec un gaz inerte tel que l'azote par exemple, et la quantité d'hydrogène utilisée est comprise de préférence dans la gamme de 2 à 200 moles pour 1 mole de NAU. Ceci présente l'avantage, lorsque de l'ammoniac est présent dans le système réactionnel, de supprimer la production d' aminé secondaire. La quantité d'ammoniac utilisée est de préférence dans la gamme de 1 à 100 moles pour 1 mole de NAU.

La réaction d'hydrogénation peut être effectuée en mode batch ou continu, mais est de préférence réalisée en continu. En mode continu, on utilise avantageusement un réacteur dans lequel le catalyseur est apporté par des flux opposés ou parallèles aux flux de matériaux NAU et d'hydrogène, de préférence le catalyseur est en lit fixe.

Le catalyseur d'hydrogénation préparé comme décrit ci-dessus conserve un niveau d'activité élevé dans une réaction en continu sur une longue période de temps et l'AA est obtenu avec un rendement élevé de 98% ou plus. De plus, comme la résistance mécanique du catalyseur est élevée il n'y a pratiquement pas de désintégration ni de désagrégation (par opposition à la plupart des catalyseurs sur charbon) du catalyseur même après avoir effectué la réaction en continu pendant de longues périodes de temps de plusieurs heures.

Selon un mode de réalisation avantageux, l'hydrogénation est réalisée en présence d'un catalyseur de métal noble et d'une substance chimique ayant un ligand polydenté qui forme un chélate par liaison avec des ions métalliques est ajouté au système de réaction. La substance chimique précitée ajoutée avec le catalyseur de métal noble forme un chélate soluble dans l'eau avec des métaux lourds tels que le fer et le nickel principalement, et elle peut être choisi parmi : des acides polyaminocarboxyliques, tels que l'acide éthylène diamine tétraacétique (EDTA), les acides hydroxycarboxyliques tels comme l'acide citrique, et les phosphates condensés, etc. La quantité de cette substance chimique ajoutée est comprise dans la gamme de 0,001 à 0.1 parts en poids pour 100 parts en poids de la charge liquide alimentée dans le réacteur d'hydrogénation (incluant le catalyseur), et de préférence de 0,002 à 0,01 parts en poids. En outre, la substance chimique précitée peut être ajoutée sous la forme d'une solution aqueuse ou sous forme d'un solide ajouté à l'alimentation en liquide. Ce mode de réalisation particulier d'hydrogénation permet au catalyseur de conserver une activité élevée lors de la réaction lorsque celle-ci est poursuivie pendant une longue période, la quantité de métaux lourds et de cendres contenus dans le produit de réaction d'hydrogénation est ainsi considérablement diminuée, et il n'y a pas de coloration notable du produit de réaction.

L'aminoacide saturé AA ainsi obtenu selon le procédé de l'invention, peut être purifié si on le souhaite, de préférence par recristallisation dans un solvant approprié.

Dans ce cas, le procédé de l'invention comprend en outre après l'étape d'hydrognation, une étape de purification de l'AA, de manière à éliminer les impuretés, notamment les impuretés amines secondaires en dessous d'un seuil < 0,5% en poids, de préférence < 0,2% en poids, sur le poids d'AA.

Tout moyen de purification adapté est utilisable dans cette étape. On peut citer notamment le procédé de purification par cristallisation. Les AA obtenus selon le procédé, dont le nombre de carbone est supérieur à 9 sont en particulier aisément recristallisables dans tout solvant convenable. Un tel procédé est décrit par exemple dans le document de brevet FR1574471 de la société BP CHEMICALS.

Avantageusement, le procédé de l'invention utilise une purification selon un procédé dans lequel le produit d'hydrogénation du NAU est recristallisé dans une solution aqueuse contenant un alcool aliphatique inférieur et de l'ammoniac. Ce procédé est notamment décrit dans le document de brevet JP48-6445 B.

Avantageusement, la purification de l'AA est réalisée par cristallisation et comprend de préférence au moins deux étapes successives de cristallisation :
- (A) une étape dans laquelle des cristaux bruts d'AA sont isolés, après dissolution du produit d'hydrogénation (AA obtenu) dans une solution aqueuse contenant un alcool aliphatique inférieur et une solution aqueuse contenant de l'ammoniac, dans un premier dispositif de cristallisation maintenu à une température comprise dans la gamme de 0-30 ° C, et
- (B) une étape dans laquelle des cristaux purs d'AA sont isolés, après redissolution des cristaux bruts d'AA obtenus à l'étape A dans une solution aqueuse contenant un alcool aliphatique inférieur et de l'ammoniac dans un deuxième dispositif de cristallisation maintenu à une température comprise dans la gamme de 30-60 ° C.

Chacune des étapes du processus de la présente invention est expliquée en termes spécifiques ci-dessous.

### L'étape (A) :

Le produit d'hydrogénation (AA), peut contenir comme sous-produits : notamment des amines secondaires et des réactifs NAU n'ayant pas réagi. Par conséquent, dans le but d'éliminer ces impuretés l'AA est dissout dans une solution aqueuse contenant un alcool aliphatique inférieur et de l'ammoniac, puis est introduit dans une première cuve de cristallisation de température dans la gamme de 0-30 ° C, puis on sépare les cristaux bruts d'AA qui contiennent principalement l'AA, et la liqueur-mère qui contient les amines secondaires et NAU. Comme alcool aliphatique inférieur utilisé dans la solution aqueuse, on utilise un alcool à chaîne linéaire ou à chaîne ramifiée en C1-4, tel que le n-propanol, l'isopropanol, le n-butanol, ou encore l'isobutanol par exemple. Le rapport en volume de l'alcool aliphatique inférieur par rapport à l'eau est de préférence compris entre 1:10-10:1, et de préférence dans la plage de 1:3-3:1. Le ratio d'ammoniac par rapport mélange d'alcool inférieur et d'eau peut varier dans un large intervalle, par exemple de 1% en poids à saturation. En outre, la concentration du produit contenant l'AA par rapport à la solution aqueuse contenant de l'alcool aliphatique inférieur et de l'ammoniac est de préférence dans la gamme de 1-10% en poids, et de manière particulièrement préférée dans la plage de 2-8% en poids.

En maintenant la température de la première cuve de cristallisation à 0-30 ° C, (qui est une température plus basse que la deuxième cuve de cristallisation ci-après), la solution est séparée en cristaux bruts qui contiennent principalement l'AA et une liqueur mère qui contient des amines secondaires et du NAU.

Les cristaux bruts d'AA sont transmis à l'étape suivante (B).

Par ailleurs, du NAU frais est ajouté à la liqueur mère, laquelle est ensuite dissoute et hydrogénée, puis recyclée dans le 1er dispositif (cuve) de cristallisation susmentionné.

### L'étape (B) :

Les cristaux brut d'AA précités et isolés à l'étape A peuvent encore contenir des impuretés, en très faibles quantités. Les cristaux brut d'AA sont donc dissous dans une solution aqueuse contenant un alcool aliphatique inférieur et de l'ammoniac, puis introduits dans un deuxième réservoir de cristallisation, puis séparés à une température de 30-60 °C en cristaux purs d'AA d'une part et une liqueur mère qui contient les amines secondaires et le NAU d'autre part.

Comme solution aqueuse contenant un alcool aliphatique inférieur et de l'ammoniac, une solution aqueuse semblable à celle utilisée dans l'étape A peut être utilisée. On peut également utiliser le même ratio d'AA par rapport à la solution aqueuse que pour l'étape (A). On peut utiliser comme solution aqueuse contenant un alcool aliphatique inférieur et d'ammoniac, une solution fraîchement préparée ou de la liqueur mère isolée dans la deuxième cuve de cristallisation peut être recyclée et réutilisée.

La température de la deuxième cuve de cristallisation est maintenue à une température plus élevée que la première cuve de cristallisation, c'est-à-dire une température de 30-60 ° C, de sorte que le reste d'impuretés présent dans les cristaux bruts issus de la première cristallisation est transférée à la liqueur mère. Par conséquent, les cristaux purs d'AA obtenus après la séparation du deuxième réservoir de cristallisation présentent une grande pureté, et l'utilisation de cristallisation à haute température, permet d'augmenter la croissance et la taille des cristaux, ce qui facilite la séparation. La liqueur mère obtenue à l'issue de l'étape B peut être redistribuée, respectivement, au 1er dispositif de cristallisation et/ou au deuxième dispositif de cristallisation comme solvant pour l'AA.

L'enchaînement d'étapes du procédé de l'invention décrit ci-dessus permet de faciliter l'étape de purification de l'AA, et facilité en particulier :
- l'élimination du réactif initial de métathèse par distillation,
- l'élimination du coproduit,
- l'élimination du catalyseur de métathèse,
- l'élimination de l'amine secondaire,
- l'hydrolyse, donc l'élimination de la source de méthanol conduisant aux réactions de N-méthylation, et
- le produit final obtenu n'est pas coloré.

De plus, l'aminoacide produit par le procédé de l'invention est directement utilisable dans les unités de polymérisation existantes ; alors que pour utiliser un aminoester il faudrait des lignes dédiées liées à l'inflammabilité du méthanol par exemple.

La purification selon le procédé de la présente invention permet de cristalliser et d'isoler de l'AA encore plus pur à partir du produit d'hydrogénation.

En effet, la teneur en impuretés, telles que les métaux lourds, dans le produit final obtenu par purification classique par cristallisation est considérablement diminuée (passée de 0,3-0,5 ppm à 0,1 ppm ou moins), et la teneur en cendres (résidus inorganiques) est considérablement diminuée (passée de 3-6 ppm à 1-1,5 ppm).

Avantageusement, le procédé de la présente invention comprend en outre une étape de synthèse de polymère, notamment de polyamide, par polymérisation utilisant l'aminoacide obtenu à l'issue de l'étape d'hydrogénation ou à l'issue de l'étape de purification. Des degrés de polymérisation bien supérieurs à 30 sont obtenus selon ce procédé. L'aminoacide produit selon le procédé de l'invention, de même que le polymère fabriqué à partir de ce monomère, ont en outre l'avantage d'être des produits incolores.

La présente divulgation a également pour objet un polymère obtenu par polymérisation utilisant l'aminoacide synthétisé selon le procédé décrit ci-dessus.

Les polymères ainsi obtenus selon le procédé de l'invention ont une viscosité élevée à l'état fondu, et il n'y a pas de coloration notable.

### EXEMPLES

### Exemple 1 (non conforme à l'invention)

### Voie métathèse-hydrogénation

Dans un réacteur en verre de 250 ml purgé à l'azote, on introduit 15g de 9-décénoate de méthyle (81 mmol) préparé selon l'exemple 1 du brevet US2011/0113679 et purifié sur alumine, 2,15g d'acrylonitrile (40,5 mmol) et 150g de toluène préalablement séché sur tamis moléculaire. On chauffe à 110°C et on introduit, via des seringues et des pousse-seringues, sur une période de 2 heures, 2,58 g d'acrylonitrile (48,6 mmol) et 2mg de catalyseur M71-SiPr (2,4.10-3 mmol, fourni par la société Umicore) dissous dans 5 ml de toluène.

Le mélange réactionnel est analysé par CPG. La conversion du 9-décénoate de méthyle est de 85%. La sélectivité en nitrile-ester insaturé en C11 est de 80%.

Le mélange réactionnel de métathèse est transféré dans un autoclave de 300 ml. On introduit dans l'autoclave 1,5g de nickel de Raney lavé au méthanol. L'autoclave est purgé à l'azote, puis on introduit 1g d'ammoniac (59 mmol) et on pressurise sous 40 bars d'hydrogène. On chauffe à 90°C et on laisse réagir 4 heures.

Le mélange réactionnel est analysé par CPG. La conversion du nitrile-ester insaturé en C11 est de 100%.

Le mélange réactionnel est filtré et le toluène est évaporé sous vide, puis on distille le résidu. On obtient ainsi 7,1g d'amino-ester en C11 (température d'ébullition=126-130°C sous 3 mbars). Le rendement de la distillation est de 60%.

Cet exemple montre que le rendement est pénalisé par un début de polymérisation de l'amino-ester pendant la distillation.

### Exemple 2 (non conforme à l'invention)

### Voie métathèse-hydrogénation-hydrolyse

Le mélange réactionnel de métathèse issu de l'exemple 1 est évaporé sous vide pour éliminer le toluène puis distillé sous vide pour récupérer le nitrile-ester en C11 (température d'ébullition=122°C sous 0,8 mbar).

Dans un autoclave de 300 ml, on charge 10g de nitrile-ester insaturé en C11 (47,8 mmol), 100g de toluène et 1g de nickel de Raney lavé au méthanol. L'autoclave est purgé à l'azote, puis on introduit 1g d'ammoniac (59 mmol) et on pressurise sous 40 bars d'hydrogène. On chauffe à 90°C et on laisse réagir 4 heures.

Le mélange réactionnel est analysé par CPG. La conversion du nitrile-ester insaturé en C11 est de 100%.

Le mélange réactionnel d'hydrogénation est filtré, évaporé sous vide pour éliminer le toluène puis transféré dans un réacteur contenant 100g d'eau. On chauffe à reflux pendant 24 heures. Le mélange réactionnel est ensuite concentré sous vide, filtré à 50°C sur charbon actif, puis refroidit. On obtient 2,9 g d'acide 11-amino-undécanoïque sous forme de cristaux blancs (rendement=30%).

Cet exemple montre que la réaction d'hydrolyse sur l'amino-ester est peu efficace.

### Exemple 3 (non conforme à l'invention)

### Voie hydrolyse-métathèse-hydrogénation

Le 9-décénoate de méthyle est transformé en acide 9-décénoïque. Dans un réacteur de 500ml, on charge 30g de 9-décénoate de méthyle (0,16 mol) et 160 ml de soude 1M (0,16 mol). On chauffe à 50°C pendant 1h. On laisse revenir à température ambiante, puis on ajoute 160 ml d'acide chlorhydrique 1M (0,16 mol). On extrait deux fois avec 300 ml de dichlorométhane. Le dichlorométhane est évaporé et on récupère 26,3g d'acide 9-décénoïque.

Dans un réacteur en verre de 250 ml purgé à l'azote, on introduit 15g d'acide 9-décénoïque (88 mmol) purifié sur alumine, 2,33g d'acrylonitrile (44 mmol) et 150g de toluène préalablement séché sur tamis moléculaire. On chauffe à 110°C et on introduit, via des seringues et des pousse-seringues, sur une période de 2 heures, 2,8g d'acrylonitrile (52,8 mmol) et 2,2mg de catalyseur M71-SiPr (2,4.10-3 mmol) dissous dans 5 ml de toluène.

Le mélange réactionnel est analysé par CPG. La conversion de l'acide 9-décénoïque est de 30%.

Cet essai montre que la réaction de métathèse est bien moins efficace sur l'acide 9-décénoïque que sur le 9-décénoate de méthyle.

### Exemple 4

### Métathèse 9-décénoate de méthyle-acrylonitrile

Dans un réacteur en verre de 250 ml purgé à l'azote, on introduit 15g de 9-décénoate de méthyle (81 mmol) purifié sur alumine, 2,15g d'acrylonitrile (40,5 mmol) et 150g de toluène préalablement séché sur tamis moléculaire. On chauffe à 110°C et on introduit, via des seringues et des pousse-seringues, sur une période de 2 heures, 2,58 g d'acrylonitrile (48,6 mmol) et 2mg de catalyseur M71-SiPr (2,4.10-3 mmol, fourni par la société Umicore) dissous dans 5 ml de toluène.

Le mélange réactionnel est analysé par CPG. La conversion du 9-décénoate de méthyle est de 85%. La sélectivité en nitrile-ester insaturé en C11 est de 80%.

Le toluène est évaporé sous vide, puis le résidu est distillé sous vide. On obtient 11,2g de nitrile-ester insaturé en C11 (53,5 mmol, température d'ébullition=122°C sous 0,8 mbar).

### Exemple 5

### Hydrolyse du nitrile-ester

Dans un réacteur en verre de 250 ml purgé à l'azote, on introduit 10g de nitrile-ester insaturé en C11 (47,8 mmol) et 100g d'un mélange acide acétique-eau 50/50. On chauffe à reflux pendant 4 heures.

Le mélange réactionnel est analysé par CPG. La conversion du nitrile-ester est de 95%. La sélectivité en nitrile-acide insaturé en C11 est de 100%. Le produit est récupéré par évaporation de l'acide acétique, du méthanol et de l'eau.

### Exemple 6

### Hydrogénation du nitrile-acide

Le catalyseur ruthénium sur carbure de silicium utilisé pour l'étape d'hydrogénation est préparé selon l'exemple 1 du brevet JP 51-127022.

Dans un autoclave de 300 ml, on introduit dans l'autoclave 5g de nitrile-acide insaturé en C11 (25,6 mmol), 100g d'une solution 50/50 de n-propanol et d'ammoniaque à 20% et 3g de catalyseur Ru/SiC. L'autoclave est fermé, purgé à l'azote, puis pressurisé sous 40 bars d'hydrogène. On chauffe à 110°C et on laisse réagir 2 heures. On dépressurise et on filtre le catalyseur à 70°C. Le solvant est évaporé sous vide et l'acide 11-aminoundécanoïque précipite sous forme de cristaux blancs. La conversion est supérieure à 95% et la sélectivité est supérieure à 95%. La teneur en amine secondaire est inférieure à 0,5%

## Revendications

1. Procédé de synthèse d'un aminoacide à partir d'un composé I gras insaturé de formule :
R1-CH=CH-[(CH₂)_{q}-CH=CH]ₚ -(CH₂)ₙ-R2
dans laquelle :
R1 est H, un radical alkyle de 1 à 11 atomes de carbone comportant le cas échéant une fonction hydroxyle, ou (CH₂)ₘ-R4
m est un indice entier compris dans la gamme de 0 à 11,
n est un indice entier compris dans la gamme de 2 à 13,
p est un indice entier, p étant égal à 0, 1 ou 2,
q est un indice égal à 0 ou 1,
R2 est COOR5 ou CN,
R4 est H ou R2
R5 est un radical alkyle de 1 à 11 atomes de carbone ou un radical comportant deux ou trois atomes de carbone porteur de une ou deux fonction(s) hydroxyle(s), ou encore le reste d'un di-glycéride ou d'un tri-glycéride dans lequel chaque acide gras dudit reste de glycéride est indifféremment saturé ou insaturé,
**caractérisé en ce qu'**il comprend au moins les étapes suivantes :
- métathèse croisée avec un composé II court insaturé, dont la chaine principale comprend moins de 8 carbones,
l'un des composés I ou II comportant une fonction nitrile et l'autre de ces composés II ou I une fonction ester, de façon à obtenir et récupérer au moins un nitrile-ester mono-unsaturé NEU,
- hydrolyse du NEU en nitrile acide insaturé NAU
- hydrogénation du NAU en AA saturé
- purification éventuelle de l'AA.

2. Procédé selon la revendication 1, dans lequel le composé I est choisi parmi les esters d'acides gras ou les nitriles dérivés d'acides gras, choisis parmi : les acides obtusilique (cis-4-décénoïque) et caproléique (9-décénoïque), les acides en C12, les acides lauroléique (cis-5-dodécénoïque) et lindérique (cis-4-dodécénoïque), les acides en C14, les acides myristoléique (cis-9-tétradécénoïque), physétérique (cis-5-tetradécénoïque) et tsuzuique (cis-4-tetradécénoïque), l'acide en C16, l'acide palmitoléique (cis-9- hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoïque) et ricinoléique (12-hydroxy--cis-9-octadécénoïque), les acides en C20, les acides gadoléique (cis-9-eicosénoïque), gondoïque (cis-11-eicosénoïque), l'acide cis-5-eicosènoïque et l'acide lesquérolique (14-hydroxy-cis-11-eicosénoïque), les acides en C22, les acides cétoléique (cis-11-docosénoïque) et érucique (cis-13-docosénoïque) ainsi que les acides polyinsaturés linoléique et linolénique.

3. Procédé selon la revendication 1 ou 2, dans lequel le composé I est choisi parmi :
CH2=CH-(CH2)ₙ-R2,
CH3-CH=CH-(CH2)ₙ-R2, ou
CH3-CH2-CH=CH-(CH2)ₙ-R2,
et de préférence le compose I est CH2=CH-(CH₂)ₙ-R2.

4. Procédé selon l'une quelconque des revendications précédentes, dans lequel R2 est COOR5, le composé II nitrile court insaturé étant de préférence choisi parmi : l'acrylonitrile, le fumaronitrile, le 2-butènenitrile, le 1-butènenitrile, le 2-pentènenitrile, le 3-pentène nitrile, le 4-pentènenitrile, le 1-pentènenitrile, et leurs mélanges.

5. Procédé selon l'une quelconque des revendications 1 à 3, dans lequel R2 est CN, le composé II ester court insaturé étant de préférence choisi parmi les composés de formule :
R6-HC=CH-(CH2)n-COOR7
dans laquelle n est 0 ou 1 ; R6 est CH3 ou H ; R7 est Me, Et ou Bu.

6. Procédé selon l'une quelconque des revendications précédentes, dans lequel l'étape de métathèse croisée utilise au moins un catalyseur ruthénium-carbène choisis parmi les catalyseurs chargés ou non-chargés de formule générale :
(X₁)ₐ (X₂)_{b}Ru(carbène C) (L₁)_{c}(L₂)_{d} (L₃)ₑ
dans laquelle :
- a, b, c, d et e sont des nombres entiers, identiques ou différents, avec a et b égaux à 0, 1 ou 2 ; c,d et e égaux à 0, 1, 2, 3 ou 4;
- X₁ et X₂, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés ; X₁ ou X₂ peuvent être liés à L₁ ou L₂ ou au carbène C de façon à former un ligand bidenté ou chélate sur le ruthénium ; et
- L₁, L₂ et L₃ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; L₁, L₂ ou L₃ peuvent être liés au carbène C de façon à former un ligand bidenté ou chélate, ou tridenté.

7. Procédé selon l'une quelconque des revendications précédentes **caractérisé par** l'utilisation d'un catalyseur de formule :

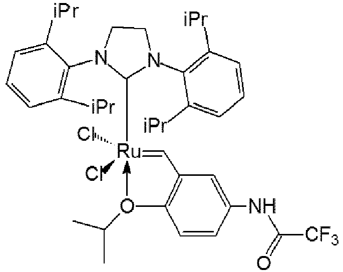

8. Procédé selon l'une quelconque des revendications précédentes **caractérisé en ce que** l'étape de métathèse est conduite en milieu liquide à une température comprise dans la gamme de 20 à 160 °C et sous une pression comprise dans la gamme de 1 à 30 bars, de préférence à une température comprise dans la gamme de 20 à 120 °C et sous une pression comprise dans la gamme de 1 à 10 bars.

9. Procédé selon l'une quelconque des revendications précédentes **caractérisé en ce que** la métathèse est conduite en présence d'un solvant, tel que : toluène, xylènes, dichlorométhane, diméthylcarbonate et/ou diéthylcarbonate.

10. Procédé selon l'une quelconque des revendications précédentes, dans lequel l'étape d'hydrolyse comprend au moins l'un des procédés suivants :
- hydrolyse basse température en présence de soude, par saponification ;
- hydrolyse à température moyenne en milieu solvant et sous catalyse acide ;
- hydrolyse haute température et sous pression ;
- hydrolyse enzymatique,
et leurs mélanges.

11. Procédé selon l'une quelconque des revendications précédentes, dans lequel l'étape d'hydrogénation est réalisée en présence d'au moins un catalyseur métallique choisi parmi le ruthénium, le rhodium, le palladium et le platine supporté sur un support carbure de silicium.

12. Procédé selon l'une quelconque des revendications précédentes, dans lequel la température d'hydrogénation est comprise dans la gamme de 10 à 300°C, et la pression est comprise dans la gamme de 1 bar à 300 bars.

13. Procédé selon l'une quelconque des revendications précédentes, dans lequel, l'étape d'hydrogénation est réalisée en présence d'un solvant comprenant un mélange d'un alcool inférieur et d'eau.

14. Procédé selon l'une quelconque des revendications précédentes, dans lequel l'hydrogénation est réalisée en présence d'un catalyseur de métal noble et d'une substance chimique ayant un ligand polydenté, ladite substance chimique étant de préférence choisie parmi: des acides polyaminocarboxyliques, tels que l'acide éthylène diamine tétraacétique (EDTA), les acides hydroxycarboxyliques tels que l'acide citrique, et les phosphates condensés.

15. Procédé selon l'une quelconque des revendications précédentes, dans lequel la purification comprend au moins une étape de recristallisation du produit issu de l'hydrogénation dans une solution aqueuse contenant un alcool aliphatique inférieur et de l'ammoniac.

16. Procédé selon l'une quelconque des revendications précédentes, dans lequel la purification comprend au moins deux étapes successives de cristallisation :
- (A) une étape dans laquelle des cristaux bruts d'AA sont isolés, après dissolution du produit d'hydrogénation dans une solution aqueuse contenant un alcool aliphatique inférieur et une solution aqueuse contenant de l'ammoniac, dans un premier dispositif de cristallisation maintenu à une température comprise dans la gamme de 0 à 30 °C, et
- (B) une étape dans laquelle des cristaux purs d'AA sont isolés, après redissolution des cristaux bruts d'AA obtenus à l'étape A dans une solution aqueuse contenant un alcool aliphatique inférieur et de l'ammoniac dans un deuxième dispositif de cristallisation maintenu à une température comprise dans la gamme de 30 à 60 ° C.

17. Procédé selon l'une quelconque des revendications 1 à 16 comprenant en outre une étape de synthèse de polyamide par polymérisation utilisant l'aminoacide.

## Patentansprüche

1. Verfahren zur Synthese einer Aminosäure aus einer ungesättigten Fettverbindung I der Formel:
R1-CH=CH-[(CH₂)_{q}-CH=CH]ₚ-(CH₂)ₙ-R2,
wobei
R1 für H, einen Alkylrest mit 1 bis 11 Kohlenstoffatomen, der gegebenenfalls eine Hydroxylfunktion enthält, oder (CH₂)ₘ-R4 steht,
m für einen ganzzahligen Index im Bereich von 0 bis 11 steht,
n für einen ganzzahligen Index im Bereich von 2 bis 13 steht,
p für einen ganzzahligen Index steht, wobei p gleich 0, 1 oder 2 ist,
q für einen Index mit einem Wert von 0 oder 1 steht,
R2 für COOR5 oder CN steht,
R4 für H oder R2 steht,
R5 für einen Alkylrest mit 1 bis 11 Kohlenstoffatomen oder einen Rest mit 2 oder 3 Kohlenstoffatomen, die eine oder zwei Hydroxylfunktionen tragen, oder auch einen Rest eines Diglycerids oder Triglycerids steht, wobei jede Fettsäure des Glyceridrests entweder gesättigt oder ungesättigt ist,
**dadurch gekennzeichnet, dass** es mindestens die folgenden Schritte umfasst:
- Kreuzmetathese mit einer ungesättigten kurzen Verbindung II, deren Hauptkette mindestens 8 Kohlenstoffatome umfasst,
wobei eine der Verbindungen I oder II eine Nitrilfunktion umfasst und die andere dieser Verbindungen II oder I eine Esterfunktion umfasst, zum Erhalt und zur Gewinnung mindestens eines einfach ungesättigten Nitrilesters UNE,
- Hydrolyse des UNE zu einer ungesättigten Nitrilsäure UNS,
- Hydrierung der UNS zu einer gesättigten AS,
- gegebenenfalls Reinigung der AS.

2. Verfahren nach Anspruch 1, wobei die Verbindung I aus Fettsäureestern oder von Fettsäureestern abgeleiteten Nitrilen, ausgewählt aus: Obtusilinsäure (cis-4-Decensäure) und Caproleinsäure (9-Decensäure), den C12-Säuren Lauroleinsäure (cis-5-Dodecensäure) und Linderinsäure (cis-4-Dodecensäure), den C14-Säuren Myristoleinsäure (cis-9-Tetradecensäure), Physeterinsäure (cis-5-Tetradecensäure) und Tsuzuinsäure (cis-4-Tetradecensäure), der C16-Säure Palmitoleinsäure (cis-9-Hexadecensäure), den C18-Säuren Ölsäure (cis-9-Octadecensäure), Elaidinsäure (trans-9-Octadecensäure), Petroselinsäure (cis-6-Octadecensäure), Vaccensäure (cis-11-Octadecensäure) und Ricinoleinsäure (12-Hydroxy-cis-9-octadecensäure), den C20-Säuren Gadoleinsäure (cis-9-Eicosensäure), Gondosäure (cis-11-Eicosensäure), cis-5-Eicosensäure und Lesquerolsäure (14-Hydroxy-cis-11-eicosensäure), den C22-Säuren Cetoleinsäure (cis-11-Docosensäure) und Erucasäure (cis-13-Docosensäure) sowie den mehrfach ungesättigten Säuren Linolsäure und Linolensäure, ausgewählt wird.

3. Verfahren nach Anspruch 1 oder 2, bei dem die Verbindung I aus:
CH2=CH-(CH2)ₙ-R2,
CH3-CH=CH-(CH2)ₙ-R2 oder
CH3-CH2-CH=CH-(CH2)ₙ-R2
ausgewählt wird und es sich bei der Verbindung I vorzugsweise um CH2=CH-(CH2)ₙ-R2 handelt.

4. Verfahren nach einem der vorhergehenden Ansprüche, wobei R2 für COOR5 steht, wobei die ungesättigte kurze Nitrilverbindung II vorzugsweise aus Acrylsäurenitril, Fumarsäurenitril, 2-Butennitril, 1-Butennitril, 2-Pentennitril, 3-Pentennitril, 4-Pentennitril, 1-Pentennitril und Mischungen davon ausgewählt wird.

5. Verfahren nach einem der Ansprüche 1 bis 3, wobei R2 für CN steht, wobei die ungesättigte kurze Esterverbindung II vorzugsweise aus den Verbindungen der Formel:
R6-HC=CH-(CH2)n-COOR7
ausgewählt wird, wobei n für 0 oder 1 steht; R6 für CH3 oder H steht; R7 für Me, Et oder Bu steht.

6. Verfahren nach einem der vorhergehenden Ansprüche, bei dem im Kreuzmetatheseschritt mindestens ein Ruthenium-Carben-Katalysator verwendet wird, der aus den geladenen oder ungeladenen Katalysatoren der allgemeinen Formel:
(X₁)ₐ(X₂)_{b}Ru(Carben C) (L₁)_{c}(L₂)_{d}(L₃)ₑ
ausgewählt wird, wobei:
- a, b, c, d und e für gleiche oder verschiedene ganze Zahlen stehen, wobei a und b gleich 0, 1 oder 2 sind; c, d und e gleich 0, 1, 2, 3 oder 4 sind;
- X₁ und X₂ gleich oder verschieden sind und jeweils für einen geladenen oder ungeladenen Mono- oder Multichelatliganden stehen; als Beispiele Halogenide, Sulfat, Carbonat, Carboxylate, Alkoholate, Phenolate, Amide, Tosylat, Hexafluorophosphat, Tetrafluoroborat, Bis(triflyl)amid, ein Alkyl, Tetraphenylborat und Derivate genannt seien; X₁ oder X₂ an L₁ oder L₂ oder an das Carben C gebunden sein können, so dass sich ein zweizähniger Ligand oder Chelatligand am Ruthenium ergibt; und
- L₁, L₂ und L₃ gleich oder verschieden sind und für elektronenliefernde Liganden wie Phosphin, Phosphit, Phosphonit, Phosphinit, Arsin, Stilbin, ein Olefin oder einen Aromaten, eine Carbonylverbindung, einen Ether, einen Alkohol, ein Amin, ein Pyridin oder Derivat, ein Imin, einen Thioether oder ein heterocyclisches Carben stehen; L₁, L₂ und L₃ an das Carben C gebunden sein können, so dass sich ein zweizähniger Ligand oder Chelatligand oder ein dreizähniger Ligand ergibt.

7. Verfahren nach einem der vorhergehenden Ansprüche, **gekennzeichnet durch** die Verwendung eines Katalysators der Formel:

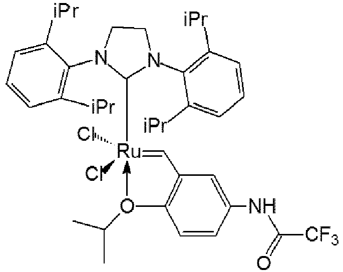

8. Verfahren nach einem der vorhergehenden Ansprüche, **dadurch gekennzeichnet, dass** der Metatheseschritt in einem flüssigen Medium bei einer Temperatur im Bereich von 20 bis 160 °C und unter einem Druck im Bereich von 1 bis 30 bar, vorzugsweise bei einer Temperatur im Bereich von 20 bis 120 °C und unter einem Druck von 1 bis 10 bar, durchgeführt wird.

9. Verfahren nach einem der vorhergehenden Ansprüche, **dadurch gekennzeichnet, dass** die Metathese in Gegenwart eines Lösungsmittels wie Toluol, Xylolen, Dichlormethan, Dimethylcarbonat und/oder Diethylcarbonat durchgeführt wird.

10. Verfahren nach einem der vorhergehenden Ansprüche, bei dem der Hydrolyseschritt mindestens die folgenden Verfahren umfasst:
- Hydrolyse bei tiefer Temperatur in Gegenwart von Natriumhydroxid durch Verseifung;
- Hydrolyse bei mittlerer Temperatur in einem Lösungsmittelmedium und unter Säurekatalyse;
- Hydrolyse bei hoher Temperatur und Unterdruck;
- enzymatische Hydrolyse und Mischungen davon.

11. Verfahren nach einem der vorhergehenden Ansprüche, bei dem der Hydrierungsschritt in Gegenwart mindestens eines Metallkatalysators, der aus Ruthenium, Rhodium, Palladium und Platin auf einem Siliciumcarbid-Träger ausgewählt wird, durchgeführt wird.

12. Verfahren nach einem der vorhergehenden Ansprüche, bei dem die Hydrierungstemperatur im Bereich von 10 bis 300 °C liegt und der Druck im Bereich von 1 bar bis 300 bar liegt.

13. Verfahren nach einem der vorhergehenden Ansprüche, bei dem der Hydrierungsschritt in Gegenwart eines Lösungsmittels, das eine Mischung aus einem niederen Alkohol und Wasser umfasst, durchgeführt wird.

14. Verfahren nach einem der vorhergehenden Ansprüche, bei dem die Hydrierung in Gegenwart eines Edelmetallkatalysators und einer chemischen Substanz mit einem mehrzähnigen Liganden durchgeführt wird, wobei die chemische Substanz vorzugsweise aus Polyaminocarbonsäuren, wie Ethylendiamintetraessigsäure (EDTA), Hydroxycarbonsäuren wie Citronensäure und kondensierten Phosphaten ausgewählt wird.

15. Verfahren nach einem der vorhergehenden Ansprüche, bei dem die Reinigung mindestens einen Schritt der Umkristallisation des Produkts aus der Hydrierung in einer wässrigen Lösung, die einen niederen aliphatischen Alkohol und Ammoniak enthält, umfasst.

16. Verfahren nach einem der vorhergehenden Ansprüche, bei dem die Reinigung mindestens zwei aufeinanderfolgende Kristallisationsschritte umfasst:
- (A) einen Schritt, in dem nach Auflösung des Hydrierungsprodukts in einer wässrigen Lösung, die einen niederen aliphatischen Alkohol und eine Ammoniak enthaltende wässrige Lösung umfasst, in einer ersten Kristallisationsvorrichtung, die bei einer Temperatur im Bereich von 0 bis 30 °C gehalten wird, rohe AS-Kristalle isoliert werden, und
- (B) einen Schritt, in dem nach Wiederauflösung der in Schritt A erhaltenen rohen AS-Kristalle in einer wässrigen Lösung, die einen niederen aliphatischen Alkohol und Ammoniak enthält, in einer zweiten Kristallisationsvorrichtung, die bei einer Temperatur im Bereich von 30 bis 60 °C gehalten wird, reine AS-Kristalle isoliert werden.

17. Verfahren nach einem der Ansprüche 1 bis 16, das außerdem einen Schritt der Synthese von Polyamid durch Polymerisation unter Verwendung der Aminosäure umfasst.

## Claims

1. Process for synthesizing an amino acid from an unsaturated fatty compound I of formula:
R1-CH=CH-[(CH₂)_{q}-CH=CH]ₚ-(CH₂)ₙ-R2
in which:
R1 is H, an alkyl radical of 1 to 11 carbon atoms comprising, where appropriate, a hydroxyl function, or (CH₂)ₘ-R4
m is an integer in the range from 0 to 11,
n is an integer in the range from 2 to 13,
p is an integer, p being equal to 0, 1 or 2,
q is an integer equal to 0 or 1,
R2 is COOR5 or CN,
R4 is H or R2
R5 is an alkyl radical of 1 to 11 carbon atoms or
a radical comprising two or three carbon atoms bearing one or two hydroxyl functions, or
alternatively a diglyceride or a triglyceride residue in which each fatty acid of said glyceride residue is either saturated or unsaturated,
**characterized in that** it comprises at least the following steps:
- cross metathesis with a short unsaturated compound II, the main chain of which comprises less than 8 carbons,
one of the compounds I or II comprising a nitrile function and the other of these compounds II or I an ester function, so as to obtain and recover at least one monounsaturated nitrile ester UNE,
- hydrolysis of the UNE into an unsaturated acid nitrile UAN
- hydrogenation of the UAN into a saturated AA
- optional purification of the AA.

2. Process according to Claim 1, in which compound I is chosen from fatty acid esters or nitriles derived from fatty acids, chosen from: obtusilic acid (cis-4-decenoic acid) and caproleic acid (9-decenoic acid), C12 acids, lauroleic acid (cis-5-dodecenoic acid) and linderic acid (cis-4-dodecenoic acid), C14 acids, myristoleic acid (cis-9-tetradecenoic acid), physeteric acid (cis-5-tetradecenoic acid) and tsuzuic acid (cis-4-tetradecenoic acid), C16 acids, palmitoleic acid (cis-9-hexadecenoic acid), C18 acids, oleic acid (cis-9-octadecenoic acid), elaidic acid (trans-9-oxodecenoic acid), petroselinic acid (cis-6-octadecenoic acid), vaccenic acid (cis-11-octadecenoic acid) and ricinoleic acid (12-hydroxy-cis-9-octadecenoic acid), C20 acids, gadoleic acid (cis-9-eicosenoic acid), gondoic acid (cis-11-eicosenoic acid), cis-5-eicosenoic acid and lesquerolic acid (14-hydroxy-cis-11-eicosenoic acid), C22 acids, cetoleic acid (cis-11-docosenoic acid) and erucic acid (cis-13-docosenoic acid), and also the polyunsaturated acids linoleic acid and linolenic acid.

3. Process according to Claim 1 or 2, in which compound I is chosen from:
CH2=CH-(CH2)ₙ-R2,
CH3-CH=CH-(CH2)ₙ-R2, or
CH3-CH2-CH=CH-(CH2)ₙ-R2,
and preferably compound I is CH2=CH-(CH2)ₙ-R2.

4. Process according to any one of the preceding claims, in which R2 is COOR5, the unsaturated short nitrile compound II preferably being chosen from: acrylonitrile, fumaronitrile, 2-butenenitrile, 1-butenenitrile, 2-pentenenitrile, 3-pentenenitrile, 4-pentenenitrile and1-pentenenitrile, and mixtures thereof.

5. Process according to any one of Claims 1 to 3, in which R2 is CN, the unsaturated short ester compound II preferably being chosen from the compounds of formula:
R6-HC=CH-(CH2)n-COOR7
in which n is 0 or 1; R6 is CH3 or H; R7 is Me, Et or Bu.

6. Process according to any one of the preceding claims, in which the cross metathesis step uses at least one ruthenium-carbene catalyst chosen from the charged or uncharged catalysts of general formula:
(X₁)ₐ (X₂)_{b}Ru(carbene C) (L₁)_{c}(L₂)_{d} (L₃)ₑ
in which:
- a, b, c, d and e are integers, which may be identical or different, with a and b equal to 0, 1 or 2; c, d and e equal to 0, 1, 2, 3 or 4;
- X₁ and X₂, which may be identical or different, each represent a charged or uncharged and monochelating or polychelating ligand; by way of example, mention may be made of halides, sulfate, carbonate, carboxylates, alkoxides, phenoxides, amides, tosylate, hexafluorophosphate, tetrafluoroborate, bis(triflyl)amide, an alkyl, tetraphenylborate and derivatives; X₁ or X₂ can be bonded to L₁ or L₂ or to the carbene C so as to form a bidentate or chelate ligand on the ruthenium; and
- L₁, L₂ and L₃, which may be identical or different, are electron-donating ligands, such as phosphine, phosphite, phosphonite, phosphinite, arsine, stilbene, an olefin or an aromatic compound, a carbonyl compound, an ether, an alcohol, an amine, a pyridine or derivative, an imine, a thioether, or a heterocyclic carbene; L₁, L₂ or L₃ can be bonded to the carbene C so as to form a bidentate or chelate ligand, or a tridentate ligand.

7. Process according to any one of the preceding claims, **characterized by** the use of a catalyst of formula:

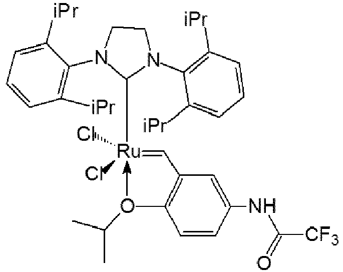

8. Process according to any one of the preceding claims, **characterized in that** the metathesis step is performed in liquid medium at a temperature in the range from 20 to 160°C and at a pressure in the range from 1 to 30 bar, preferably at a temperature in the range from 20 to 120°C and at a pressure in the range from 1 to 10 bar.

9. Process according to any one of the preceding claims, **characterized in that** the metathesis is performed in the presence of a solvent such as: toluene, xylenes, dichloromethane, dimethyl carbonate and/or diethyl carbonate.

10. Process according to any one of the preceding claims, in which the hydrolysis step comprises at least one of the following processes:
- low-temperature hydrolysis in the presence of sodium hydroxide, by saponification;
- medium-temperature hydrolysis in solvent medium and under acidic catalysis;
- high-temperature hydrolysis under pressure;
- enzymatic hydrolysis,
and mixtures thereof.

11. Process according to any one of the preceding claims, in which the hydrogenation step is performed in the presence of at least one metal catalyst chosen from ruthenium, rhodium, palladium and platinum supported on a silicon carbide support.

12. Process according to any one of the preceding claims, in which the hydrogenation temperature is in the range from 10 to 300°C and the pressure is in the range from 1 bar to 300 bar.

13. Process according to any one of the preceding claims, in which the hydrogenation step is performed in the presence of a solvent comprising a mixture of a lower alcohol and water.

14. Process according to any one of the preceding claims, in which the hydrogenation is performed in the presence of a noble metal catalyst and of a chemical substance bearing a polydentate ligand, said chemical substance preferably being chosen from: polyaminocarboxylic acids, such as ethylenediaminetetraacetic acid (EDTA), hydroxycarboxylic acids such as citric acid, and condensed phosphates.

15. Process according to any one of the preceding claims, in which the purification comprises at least one step of recrystallization of the product derived from the hydrogenation in an aqueous solution containing a lower aliphatic alcohol and ammonia.

16. Process according to any one of the preceding claims, in which the purification comprises at least two successive crystallization steps:
- (A) a step in which crude AA crystals are isolated, after dissolution of the hydrogenation product in an aqueous solution containing a lower aliphatic alcohol and an aqueous solution containing ammonia, in a first crystallization device maintained at a temperature in the range from 0 to 30°C, and
- (B) a step in which pure AA crystals are isolated, after redissolution of the crude AA crystals obtained in step A in an aqueous solution containing a lower aliphatic alcohol and ammonia in a second crystallization device maintained at a temperature in the range from 30 to 60°C.

17. Process according to any one of Claims 1 to 16, also comprising a step of polyamide synthesis by polymerization using the amino acid.
